# EUROPEAN PATENT SPECIFICATION

(11) **EP 4 129 163 B1**
(45) Date of publication and mention of the grant of the patent: **11.09.2024**
(21) Application number: 21781584.4
(22) Date of filing: 19.02.2021
(51) Int. Cl.: A61B 5/02, A61B 5/026

(54) **BIOLOGICAL INFORMATION MEASUREMENT DEVICE, BIOLOGICAL INFORMATION MEASUREMENT SYSTEM, AND BIOLOGICAL INFORMATION MEASUREMENT METHOD**
VORRICHTUNG ZUR MESSUNG BIOLOGISCHER INFORMATIONEN, SYSTEM ZUR MESSUNG BIOLOGISCHER INFORMATIONEN UND VERFAHREN ZUR MESSUNG BIOLOGISCHER INFORMATIONEN
DISPOSITIF DE MESURE D'INFORMATIONS BIOLOGIQUES, SYSTÈME DE MESURE D'INFORMATIONS BIOLOGIQUES ET PROCÉDÉ DE MESURE D'INFORMATIONS BIOLOGIQUES

(30) Priority: 01.04.2020 JP 2020066041
(43) Date of publication of application: 08.02.2023
(73) Proprietor: Sony Group Corporation, Tokyo 108-0075 (JP)
(72) Inventor: IKUTA, Tomoya, Tokyo 108-0075 (JP); ITO, Atsushi, Tokyo 108-0075 (JP)
(74) Representative: D Young & Co LLP
(86) International application number: PCT/JP2021/006249
(87) International publication number: WO 2021/199773

(56) References cited:
- WO-A1-2018/112401
- JP-A- 2008 173 510
- JP-A- 2008 272 085
- JP-A- 2010 194 000
- JP-A- 2017 018 772
- JP-A- 2017 534 325
- JP-A- H0 576 504
- JP-B1- 5 930 012
- US-A1- 2007 135 717
- US-A1- 2016 238 443
- US-A1- 2016 310 027

## Description

### TECHNICAL FIELD

The present technology relates to a biological information measurement apparatus, a biological information measurement system, and a biological information measurement method.

### BACKGROUND ART

Currently, there are many measurement devices that acquire biological information from human bodies in the world. Among the devices, devices that measure pulses have been widely used as pulse sensors that are clock-type devices or wristband-type devices along with the development of wearable devices, and a photoplethysmography (PPG) method has been widely adopted. Furthermore, devices that measure blood flow rates have been widely used in the medical field and the like, and a laser Doppler flowmetry (LDF) and the like are known. Since pieces of biological information such as a volume pulse wave and a blood flow rate reflect a change in the cardiovascular system of the human body, the biological information is very effective in observing the state of the autonomic nerves of the human body.

Conventionally, it is known that, when the state of the autonomic nerves is observed on the basis of the biological information such as a volume pulse wave and a blood flow rate, a different behavior is exhibited for each blood vessel in a case where the state of the autonomic nerves is generally in a tension state. Therefore, a plurality of pieces of biological information is simultaneously acquired and the state of the autonomic nerves is identified on the basis of the behavior of the plurality of pieces of biological information, whereby the accuracy of the identification is improved. For example, Patent Document 1 discloses a technique in which a plurality of volume pulse waves is measured using a plurality of different detection methods, a difference between different pieces of the biological information is calculated, whereby new information is acquired and accuracy of identifying the state of the autonomic nerves is improved.

### CITATION LIST

### PATENT DOCUMENT

Patent Document 1: Japanese Patent Application Laid-Open No. 2018-68556

Other prior art is provided in US2016/238443A1 and US2007/135717A.

### SUMMARY OF THE INVENTION

### PROBLEMS TO BE SOLVED BY THE INVENTION

However, in a case where as the plurality of different detection methods, transmissive type and reflective type measurements of volume pulse waves are performed using one light source, measurement sites such as blood vessels overlap in the vicinity of the light source. Furthermore, as another detection method, there is also a method of measuring a volume pulse wave using different wavelength light sources. However, in this case, light receiving units are the same as each other, and measurement sites such as blood vessels to be measured overlap in the vicinity of the light receiving unit.

Therefore, in the case of the above-described means, there is a concern about a problem that a volume pulse wave signal has low independence and contains many pieces of biological information derived from the same measurement site and in a case where a difference between the pieces of biological information is calculated, the difference is not clear, and identification accuracy decreases.

Furthermore, there is a concern that even in a case where at least one light emitting element and at least two light receiving elements are disposed on substantially the same plane, it is assumed that different light receiving units receive light having passed through the same optical path in a living body near a light source, and biological information derived from the same measurement site may be included.

Therefore, a main object of the present technology is to provide technology capable of acquiring biological information of different independent measurement sites.

### SOLUTIONS TO PROBLEMS

That is, the present technology provides a biological information measurement apparatus according to claim 1.

### BRIEF DESCRIPTION OF DRAWINGS

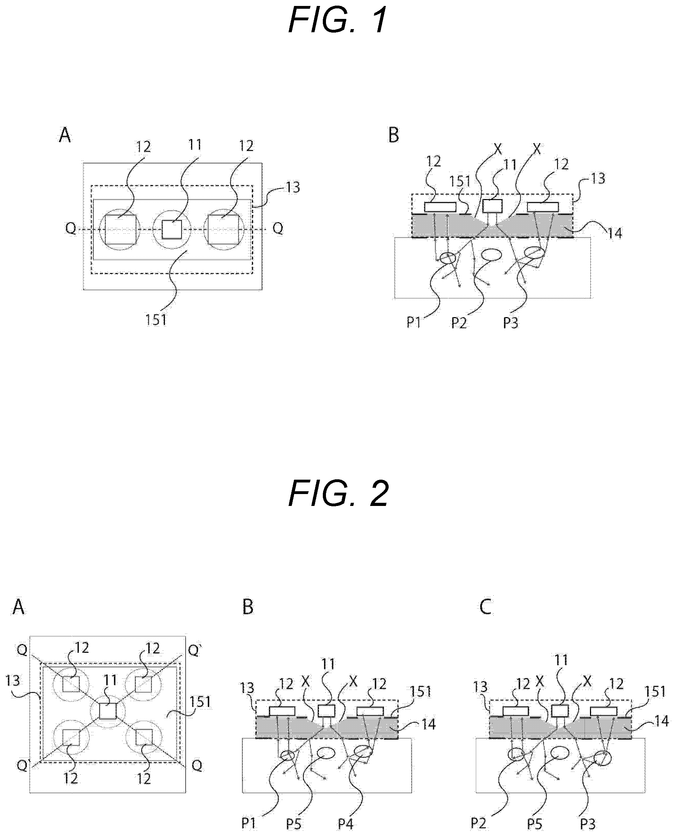

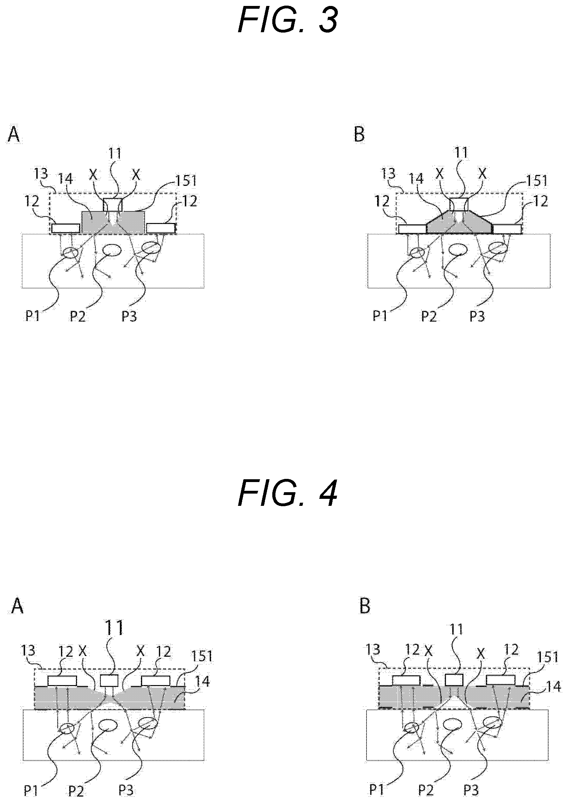

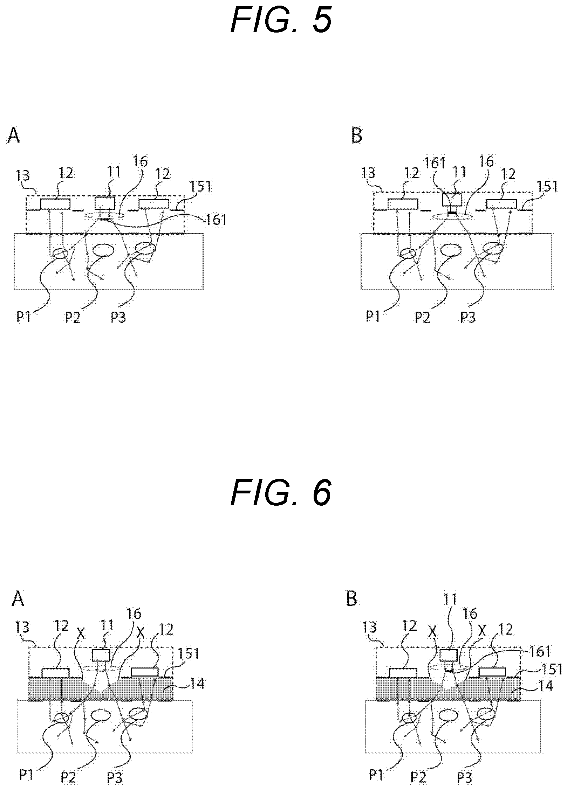

A of Fig. 1 is a schematic diagram illustrating configuration example 1, and B of Fig. 1 is a schematic cross-sectional diagram taken along line Q-Q of A of Fig. 1.
A of Fig. 2 is a schematic diagram illustrating configuration example 2, B of Fig. 2 is a schematic cross-sectional diagram taken along line Q-Q of A of Fig. 2, and C of Fig. 2 is a schematic cross-sectional diagram taken along line Q'-Q' of A of Fig. 2.
A of Fig. 3 is a schematic diagram illustrating configuration example 3, and B of Fig. 3 is a schematic diagram illustrating configuration example 4.
A of Fig. 4 is a schematic diagram illustrating configuration example 5, and B of Fig. 4 is a schematic diagram illustrating configuration example 6.
A of Fig. 5 is a schematic diagram illustrating configuration example 7, and B of Fig. 5 is a schematic diagram illustrating configuration example 8.
A of Fig. 6 is a schematic diagram illustrating configuration example 9, and B of Fig. 6 is a schematic diagram illustrating configuration example 10.
Fig. 7 is a schematic diagram illustrating configuration example 11.
Fig. 8 is a schematic diagram illustrating configuration example 12.
Fig. 9 is a block diagram illustrating configuration example of a processing unit 100.
A of Fig. 10 is a schematic diagram illustrating configuration example 13, and B of Fig. 10 is a schematic cross-sectional diagram taken along line Q-Q of A of Fig. 10.
Fig. 11 is a diagram illustrating an example of time-division driving.
A of Fig. 12 is a schematic diagram illustrating configuration example 14, B of Fig. 12 is a schematic cross-sectional diagram taken along line Q-Q of A of Fig. 12, and C of Fig. 12 is a schematic cross-sectional diagram taken along line Q'-Q' of A of Fig. 12.

### MODE FOR CARRYING OUT THE INVENTION

Hereinafter, preferred embodiments for carrying out the present technology will be described.

Note that embodiments to be described below are representative embodiments of the present technology, and the scope of the present technology is not narrowly interpreted by the embodiments. Note that description of the present technology will be given in the following order.
1. Biological Information Measurement Apparatus 10 According to First Embodiment of Present Technology
   1-1. Configurations of Biological Information Measurement Apparatus 10 According to First Embodiment
   1-2. Configuration Example 1
   1-3. Configuration Example 2
   1-4. Configuration Example 3 and Configuration Example 4
   1-5. Configuration Example 5 and Configuration Example 6
   1-6. Configuration Example 7 and Configuration Example 8
   1-7. Configuration Example 9 and Configuration Example 10
   1-8. Configuration Example 11
   1-9. Configuration Example 12
   1-10. Processing Unit 100
2. Biological Information Measurement Apparatus 10 According to Second Embodiment of Present Technology
   2-1. Configurations of Biological Information Measurement Apparatus 10 According to Second Embodiment
   2-2. Configuration Example 13
   2-3. Configuration Example 14
3. Biological Information Measurement System According to Present Technology
4. Biological Information Measurement Method According to Present Technology

### 1. Biological Information Measurement Apparatus 10 According to First Embodiment of Present Technology

Hereinafter, configurations of a biological information measurement apparatus 10 according to the present embodiment will be described. Note that the embodiment illustrates a preferred example of the present technology, and the biological information measurement apparatus 10 according to the present technology is not limited to the configurations. Furthermore, in embodiments to be illustrated below, a measurement site is not included in these embodiments.

### 1-1. Configurations of Biological Information Measurement Apparatus 10 According to First Embodiment

The biological information measurement apparatus 10 according to the present embodiment includes: a light emitting unit 11 that irradiates a living body with light; and a light receiving unit 12 that receives light scattered at a plurality of measurement sites in the living body, in which the biological information measurement apparatus includes a mechanism S in which the light scattered at the plurality of measurement sites is individually detected by a plurality of the light receiving units or the same light receiving unit.

The biological information measurement apparatus 10 according to the present embodiment is worn, for example, on part of skin of a living body (for example, human body) and can take various forms, for example, a wristband type, an earring type, a ring type, a necklace type, an attachment type, a supporter type, and the like.

In the present description, "biological information" refers to any information of the living body. The living body includes, for example, a human body and a body of as an animal or the like other than a human. In the present technology, the biological information is preferably blood vessel information of the living body. The acquisition of the blood vessel information of the living body enables, for example, identification of a state of autonomic nerves.

In the present technology, as illustrated in Fig. 1, the number of measurement sites in the living body is plural (at least two or more). In the present technology, the measurement site is preferably a blood vessel of an animal including a human.

The light emitting unit 11 is a part that irradiates the living body with light. The light emitting unit 11 includes at least one or more light emitting elements.

Examples of the light emitting element include laser light sources such as a light emitting diode (LED), an edge emitting laser (LD), and a vertical cavity surface emitting laser (VCSEL).

Furthermore, for example, if the light emitting element is a laser light source that emits coherent light, the light emitting element can be used for measuring biological information such as a volume pulse wave and a blood flow rate. Furthermore, for example, if the light emitting element is a LED that emits non-coherent light, the light emitting element can be used for measuring biological information such as a volume pulse wave and a blood flow rate. In the present technology, light emitted from the light emitting element may be visible light or invisible light (for example, infrared light).

As a wavelength of the light emitting element, for example, a wavelength in a visible region, a near-infrared region, or an infrared region can be used.

The light receiving unit 12 is a part that receives the light scattered at the plurality of measurement sites in the living body. The light receiving unit 12 includes at least one or more light receiving elements.

The light receiving element includes, for example, a photodiode (PD), a phototransistor, and the like.

Specifically, as the light receiving element, it is possible to adopt, for example, a multi-division photodiode PD having a plurality of light receiving regions disposed one-dimensionally or two-dimensionally, a line sensor in which pixels including a PD are disposed one-dimensionally, an image sensor in which pixels including a PD are disposed two-dimensionally, or the like.

The biological information measurement apparatus 10 according to the present embodiment includes the mechanism S in which the light scattered at the plurality of measurement sites is individually detected by the plurality of light receiving units or the same light receiving unit (also simply referred to as "mechanism S" in the present specification).

Thus, the provision of the mechanism S allows each optical path to pass through a different independent measurement site and allows a biological signal from the independent measurement site to be acquired. A plurality of pieces of highly independent biological information is handled, whereby it is possible to achieve the measurement of biological information, for example, the state or the like of the autonomic nerves of the living body with high identification accuracy.

The biological information measurement apparatus 10 according to the present embodiment includes at least one or more light emitting units 11 and a plurality of (at least two or more) light receiving units 12. Furthermore, a processing unit 100 to be described later may be included as necessary. Hereinafter, specific configurations of the biological information measurement apparatus 10 according to the present embodiment will be described in detail.

### 1-2. Configuration Example 1

A of Fig. 1 is a schematic diagram illustrating configuration example 1 of the mechanism S, and B of Fig. 1 is a schematic cross-sectional diagram taken along line Q-Q of A of Fig. 1. Configuration example 1 includes one light emitting unit 11 that irradiates the inside of a living body with light, two light receiving units 12 that receive light scattered in the living body and acquire a biological signal, and a housing 13 including the light emitting unit 11 and the light receiving units 12, and the one light emitting unit 11 and the two light receiving units 12 are disposed in the same straight line. Furthermore, a member 14 having a transmissive surface X through which light emitted from the light emitting unit 11 is transmitted is included.

The member 14 includes, for example, a material having a refractive index different from a refractive index of air, and examples of the material include plastic, glass, resin, metal, and the like. The member 14 is formed with these materials, whereby the light emitted from the light emitting unit 11 can be refracted.

As illustrated in B of Fig. 1, the member 14 is disposed between the light emitting unit 11 and the measurement site. In this case, the transmissive surface X is disposed to be inclined with respect to an irradiation optical path of the light emitting unit 11. With this configuration, it is possible to refract the light emitted from the light emitting unit 11, and it is possible for each light receiving unit 12 to individually acquire information of light having passed through different measurement sites (for example, blood vessels P1 and P3) without passing through the same measurement site (for example, blood vessel P2).

Configuration example 1 further includes a light shielding unit 151 on the peripheral surface of each of the light emitting unit 11 and the light receiving unit 12, the peripheral surface excluding a surface of each of the light emitting unit 11 and the light receiving unit 12 in contact with the member 14 and a surface that faces the surface and that are in contact with the living body or the like. With this configuration, it is possible to prevent stray light and improve measurement accuracy.

Examples of a material constituting the light shielding unit 151 include an alloy or organic material, and the like of silver (Ag) and at least one kind selected from titanium (Ti), tungsten (W), carbon (C), chromium oxide (Cr₂O₃), and samarium (Sm). The light shielding unit 151 is configured as, for example, a single-layer film or a stacked-layer film including these materials. Furthermore, a light shielding sheet, a light shielding filter, or the like may be attached to the light shielding unit 151. In the present embodiment, the shape of the light shielding unit 151 is not particularly limited as long as stray light can be prevented. Furthermore, the disposition of the light shielding unit 151 can be also freely designed as appropriate.

### 1-3. Configuration Example 2

A of Fig. 2 is a schematic diagram illustrating configuration example 2 of the mechanism S, B of Fig. 2 is a schematic cross-sectional diagram taken along line Q-Q of A of Fig. 2, and C of Fig. 2 is a schematic cross-sectional diagram taken along line Q'-Q' of A of Fig. 2. Configuration example 2 includes one light emitting unit 11 that irradiates the inside of a living body with light, four light receiving units 12 that receive light scattered in the living body and acquire a biological signal, and a housing 13 including the light emitting unit 11 and the light receiving units 12, and the four light receiving units 12 are disposed in the diagonal lines of the light emitting unit 11 with the one light emitting unit 11 interposed between the light receiving units 12. Furthermore, a member 14 having a transmissive surface X through which the light emitted from the light emitting unit 11 described above is transmitted is included, and a light shielding unit 151 is included on the peripheral surface of each of the light emitting unit 11 and the light receiving unit 12, the peripheral surface excluding a surface of each of the light emitting unit 11 and the light receiving unit 12 in contact with the member 14 and a surface that faces the surface and that are in contact with the living body or the like. With this configuration, it is possible to refract the light emitted from the light emitting unit 11, and it is possible for each light receiving unit 12 to individually acquire information of light having passed through different measurement sites (for example, blood vessels P1, P2, P3, and P4) without passing through the same measurement site (for example, blood vessel P5).

In the present embodiment, the member 14 preferably has a conical air layer including the transmissive surface X as illustrated in A to C of Fig. 2. With this configuration, even in a case where the number of the light receiving units 12 increases as illustrated in configuration example 2, it is possible for all the light receiving units 12 to acquire information of light having passed through different measurement sites (for example, blood vessels P1, P2, P3, and P4) without acquiring information of light having passed through the same measurement site (for example, blood vessel P5).

### 1-4. Configuration Example 3 and Configuration Example 4

A of Fig. 3 is a schematic diagram illustrating configuration example 3 of the mechanism S. Configuration example 3 includes one light emitting unit 11 that irradiates the inside of a living body with light, two light receiving units 12 that receive light scattered in the living body and acquire a biological signal, and a housing 13 including the light emitting unit 11 and the light receiving units 12, and a plurality of the light receiving units 12 is disposed on substantially the same plane. Furthermore, a member 14 having a transmissive surface X through which light emitted from the light emitting unit 11 described above is transmitted is included, and a light shielding unit 151 is included on the peripheral surface of the light emitting unit 11, the peripheral surface excluding a surface of the light emitting unit 11 in contact with the member 14 and a surface that faces the surface and that is in contact with the living body or the like. With this configuration, it is possible to refract the light emitted from the light emitting unit 11, and it is possible for each light receiving unit 12 to individually acquire information of light having passed through different measurement sites (for example, blood vessels P1 and P3) without passing through the same measurement site (for example, blood vessel P2).

Furthermore, B of Fig. 3 is a schematic diagram illustrating configuration example 4 of the mechanism S. Configuration example 4 includes one light emitting unit 11 that irradiates the inside of a living body with light, two light receiving units 12 that receive light scattered in the living body and acquire a biological signal, and a housing 13 including the light emitting unit 11 and the light receiving units 12, and a plurality of the light receiving units 12 is disposed on substantially the same plane. Furthermore, a member 14 having a transmissive surface X through which light emitted from the light emitting unit 11 described above is transmitted is included, and a light shielding unit 151 is included on the peripheral surface of the light emitting unit 11, the peripheral surface excluding a surface of the light emitting unit 11 in contact with the member 14 and a surface that faces the surface and that is in contact with the living body or the like. With this configuration, it is possible to refract the light emitted from the light emitting unit 11, and it is possible for each light receiving unit 12 to individually acquire information of light having passed through different measurement sites (for example, blood vessels P1 and P3) without passing through the same measurement site (for example, blood vessel P2).

In a case where the biological information measurement apparatus 10 according to the present technology includes a plurality of light receiving units 12, the plurality of light receiving units 12 can be disposed on substantially the same plane as illustrated in A and B of Fig. 3.

Furthermore, in the present technology, the shape of the light shielding unit 151 is not particularly limited as long as stray light can be prevented, and the light shielding unit 151 can be freely designed into a desired shape as illustrated in A and B of Fig. 3.

### 1-5. Configuration Example 5 and Configuration Example 6

A of Fig. 4 is a schematic diagram illustrating configuration example 5 of the mechanism S. Configuration example 5 includes one light emitting unit 11 that irradiates the inside of a living body with light, two light receiving units 12 that receive light scattered in the living body and acquire a biological signal, and a housing 13 including the light emitting unit 11 and the light receiving units 12, and the light emitting unit 11 and a plurality of the light receiving units 12 are disposed on substantially the same plane. Furthermore, a member 14 having a transmissive surface X through which the light emitted from the light emitting unit 11 described above is transmitted is included, and a light shielding unit 151 is included on the peripheral surface of each of the light emitting unit 11 and the light receiving unit 12, the peripheral surface excluding a surface of each of the light emitting unit 11 and the light receiving unit 12 in contact with the member 14 and a surface that faces the surface and that are in contact with the living body or the like. With this configuration, it is possible to refract the light emitted from the light emitting unit 11, and it is possible for each light receiving unit 12 to individually acquire information of light having passed through different measurement sites (for example, blood vessels P1 and P3) without passing through the same measurement site (for example, blood vessel P2).

Furthermore, B of Fig. 4 is a schematic diagram illustrating configuration example 6 of the mechanism S. Configuration example 6 includes one light emitting unit 11 that irradiates the inside of a living body with light, two light receiving units 12 that receive light scattered in the living body and acquire a biological signal, and a housing 13 including the light emitting unit 11 and the light receiving units 12, and the light emitting unit 11 and a plurality of the light receiving units 12 are disposed on substantially the same plane. Furthermore, a member 14 having a transmissive surface X through which the light emitted from the light emitting unit 11 described above is transmitted is included, and a light shielding unit 151 is included on the peripheral surface of each of the light emitting unit 11 and the light receiving unit 12, the peripheral surface excluding a surface of each of the light emitting unit 11 and the light receiving unit 12 in contact with the member 14 and a surface that faces the surface and that are in contact with the living body or the like. With this configuration, it is possible to refract the light emitted from the light emitting unit 11, and it is possible for each light receiving unit 12 to individually acquire information of light having passed through different measurement sites (for example, blood vessels P1 and P3) without passing through the same measurement site (for example, blood vessel P2).

In a case where the biological information measurement apparatus 10 according to the present technology includes a plurality of light receiving units 12, the light emitting unit 11 and the plurality of light receiving units 12 can be disposed on substantially the same plane as illustrated in A and B of Fig. 4.

Furthermore, in the present embodiment, as illustrated in A of Fig. 4, the member 14 may have a plurality of conical air layers including the transmissive surface X.

### 1-6. Configuration Example 7 and Configuration Example 8

A of Fig. 5 is a schematic diagram illustrating configuration example 7 of the mechanism S. Configuration example 7 includes one light emitting unit 11 that irradiates the inside of a living body with light, two light receiving units 12 that receive light scattered in the living body and acquire a biological signal, and a housing 13 including the light emitting unit 11 and the light receiving units 12, and the light emitting unit 11 and a plurality of the light receiving units 12 are disposed on substantially the same plane. Furthermore, a lens 16 through which the light emitted from the light emitting unit 11 is transmitted is included.

As illustrated in A of Fig. 5, the lens 16 is disposed between the light emitting unit 11 and the measurement site. Furthermore, as illustrated in A of Fig. 5, the lens 16 preferably includes a lens light shielding unit 161 in a part of the lens 16. With this configuration, it is possible to remove light in a beam center part, the light having spread after being emitted from the lens, and it is possible for each light receiving unit 12 to individually acquire information of light having passed through different measurement sites (for example, blood vessels P1 and P3) without passing through the same measurement site (for example, blood vessel P2). Note that a material constituting the lens light shielding unit 161 is similar to the material constituting the light shielding unit 151 described above.

B of Fig. 5 is a schematic diagram illustrating configuration example 8 of the mechanism S. Configuration example 8 includes one light emitting unit 11 that irradiates the inside of a living body with light, two light receiving units 12 that receive light scattered in the living body and acquire a biological signal, and a housing 13 including the light emitting unit 11 and the light receiving units 12, and the light emitting unit 11 and a plurality of the light receiving units 12 are disposed on substantially the same plane. Furthermore, a lens 16 through which the light emitted from the light emitting unit 11 is transmitted is included.

In configuration example 8, similarly to configuration example 7, the lens 16 includes a lens light shielding unit 161 in a part of the lens 16. The lens light shielding unit 161 is preferably provided at a lens center part on a side where the light emitted from the light emitting unit 11 is incident as illustrated in A of Fig. 5 or on a side where the light emitted from the light emitting unit 11 is emitted as illustrated in B of Fig. 5. With this configuration, it is possible to efficiently remove light in a beam center part, the light having spread after being emitted from the lens. As a result, it is possible to block an optical path passing through the same measurement site (for example, blood vessel P2). Furthermore, in configuration example 7 and configuration example 8, in a case where a lens that is centrosymmetric with respect to incident light is used, even in a case where the number of the light receiving units 12 increases, it is possible to cause each light receiving unit 12 to acquire information of light at different measurement sites.

Configuration examples 7 and 8 each further include a light shielding unit 151 described above that is different from the lens light shielding unit 161 on the peripheral surface of each of the light emitting unit 11 and the light receiving unit 12, the peripheral surface excluding a bottom surface of each of the light emitting unit 11 and the light receiving unit 12 on a side where the measurement site exists. With this configuration, it is possible to prevent stray light and improve measurement accuracy.

### 1-7. Configuration Example 9 and Configuration Example 10

A of Fig. 6 is a schematic diagram illustrating configuration example 9 of the mechanism S. Configuration example 9 includes one light emitting unit 11 that irradiates the inside of a living body with light, two light receiving units 12 that receive light scattered in the living body and acquire a biological signal, and a housing 13 including the light emitting unit 11 and the light receiving units 12, and a plurality of the light receiving units 12 is disposed on substantially the same plane. Furthermore, a member 14 having a transmissive surface X through which the light emitted from the light emitting unit 11 described above is transmitted is included, and a lens 16 through which the light emitted from the light emitting unit 11 is transmitted is included.

In the present embodiment, as illustrated in A of Fig. 6, both the member 14 and the lens 16 described above may be used in combination, whereby each light receiving unit 12 may be configured to individually acquire information of light having passed through different measurement sites (for example, blood vessels P1 and P3) without passing through the same measurement site (for example, blood vessel P2).

B of Fig. 6 is a schematic diagram illustrating configuration example 10 of the mechanism S. Configuration example 10 includes one light emitting unit 11 that irradiates the inside of a living body with light, two light receiving units 12 that receive light scattered in the living body and acquire a biological signal, and a housing 13 including the light emitting unit 11 and the light receiving units 12, and a plurality of the light receiving units 12 is disposed on substantially the same plane. Furthermore, a member 14 having a transmissive surface X through which the light emitted from the light emitting unit 11 described above is transmitted is included, and a lens 16 through which the light emitted from the light emitting unit 11 is transmitted is included.

In configuration example 10, unlike configuration example 9 described above, the lens 16 includes a lens light shielding unit 161. Thus, the lens light shielding unit 161 can be included as necessary.

Configuration example 9 and configuration example 10 each further include a light shielding unit 151 described above that is different from the lens light shielding unit 161 on the peripheral surface of each of the light emitting unit 11 and the light receiving unit 12, the peripheral surface excluding a bottom surface of each of the light emitting unit 11 and the light receiving unit 12 on a side where the measurement site exists (a surface of each of the light emitting unit 11 and the light receiving unit 12 in contact with the member 14 and a surface that faces the surface and that are in contact with the living body or the like). With this configuration, it is possible to prevent stray light and improve measurement accuracy.

### 1-8. Configuration Example 11

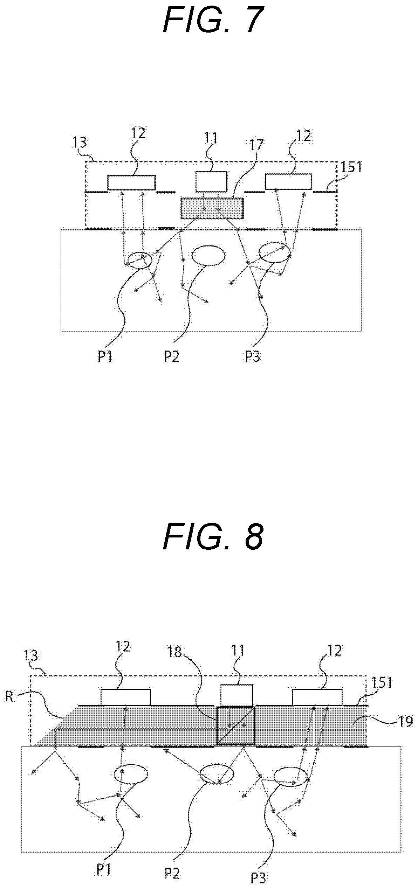

Fig. 7 is a schematic diagram illustrating configuration example 11 of the mechanism S. Configuration example 11 includes one light emitting unit 11 that irradiates the inside of a living body with light, two light receiving units 12 that receive light scattered in the living body and acquire a biological signal, and a housing 13 including the light emitting unit 11 and the light receiving units 12. Furthermore, a diffraction grating unit 17 that generates diffracted light on the basis of the light emitted from the light emitting unit 11 is included.

As illustrated in A of Fig. 7, the diffraction grating unit 17 is disposed between the light emitting unit 11 and the measurement site. With this configuration, the light emitted from the light emitting unit 11 is branched by the diffraction grating unit 17, and it is possible for each light receiving unit 12 to individually acquire information of light having passed through different measurement sites (for example, blood vessels P1 and P3) without passing through the same measurement site (for example, blood vessel P2).

The diffraction grating unit 17 can be freely designed as appropriate so that the diffraction grating unit 17 freely determines a branching direction of light. Therefore, even in a case where the number of the light receiving units 12 increases, light having passed through different measurement sites (for example, blood vessels P1 and P3) can be made incident on each of the light receiving units 12.

Configuration example 11 further includes a light shielding unit 151 described above on the peripheral surface of each of the light emitting unit 11 and the light receiving unit 12, the peripheral surface excluding a bottom surface of each of the light emitting unit 11 and the light receiving unit 12 on a side where the measurement site exists. With this configuration, it is possible to prevent stray light and improve measurement accuracy.

### 1-9. Configuration Example 12

Fig. 8 is a schematic diagram illustrating configuration example 12 of the mechanism S. Configuration example 12 includes one light emitting unit 11 that irradiates the inside of a living body with light, two light receiving units 12 that receive light scattered in the living body and acquire a biological signal, and a housing 13 including the light emitting unit 11 and the light receiving units 12. Furthermore, a light dividing unit 18 that divides the light emitted from the light emitting unit 11 described above is included.

The light dividing unit 18 is, for example, one that divides light into two and includes a half beam splitter (HBS), a branching waveguide, and the like. With this configuration, an optical path is branched into two and sites of the living body where light is incident are separated, whereby pieces of information of different measurement sites (for example, blood vessels P1 and P3) can be individually acquired by different light receiving parts 12.

In configuration example 12, a member 19 having a refractive surface R in which light divided by the light dividing unit 18 is refracted is further included. The member 19 is disposed between the light emitting unit 11 and a plurality of the measurement sites. The refractive surface R is disposed to be inclined with respect to an optical path of the light divided by the light dividing unit 18. With this configuration, it is possible to refract the light divided by the light dividing unit 18 and irradiate one (for example, blood vessel P1) of the plurality of measurement sites (for example, blood vessels P1 and P3) with light.

Similarly to the member 14 described above, the member 19 includes, for example, a material having a refractive index different from a refractive index of air, and examples of the material include plastic, glass, resin, metal, and the like.

Furthermore, a reflection surface having a high reflectance such as aluminum or gold may be added to the refractive surface R to actively reflect light.

Configuration example 12 further includes a light shielding unit 151 described above on the peripheral surface of each of the light emitting unit 11 and the light receiving unit 12, the peripheral surface excluding a bottom surface of each of the light emitting unit 11 and the light receiving unit 12 on a side where the measurement site exists. With this configuration, it is possible to prevent stray light and improve measurement accuracy.

### 1-10. Processing Unit 100

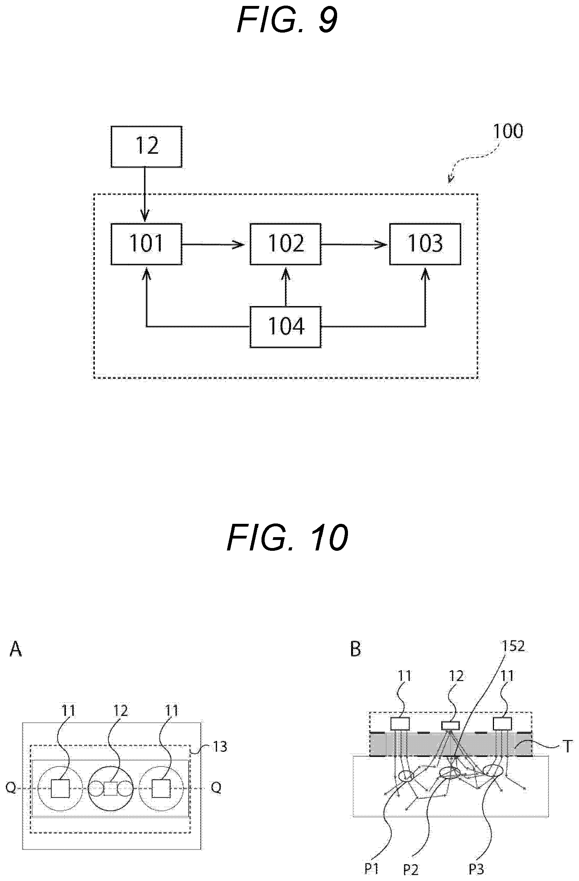

Fig. 9 is a block diagram illustrating a configuration example of the processing unit 100.

The processing unit 100 analyzes the biological information (for example, a state of the autonomic nerves) on the basis of output from the light receiving unit 12.

The processing unit 100 can include, for example, an extracting unit 101, a recording unit 102, and an analyzing unit 103. Furthermore, the processing unit 100 may include a controlling unit 104 that integrally controls each part as necessary.

The processing unit 100 may be provided in the housing 13 described above and connected to the light receiving unit 12, may be provided integrally with the housing 13 outside the housing 13 and connected to the light receiving unit 12, or may be provided outside the housing 13 and connected to the light receiving unit 12 in a wired or wireless manner.

The extracting unit 101 extracts information of a plurality of measurement sites from the output of the light receiving unit 12. The extracting unit 101 is achieved by, for example, a low-pass filter.

The low-pass filter is connected to an output end of the light receiving unit 12, cuts off a high-frequency component (noise component) of a signal output from the light receiving unit 12, and outputs a signal obtained by cutting off the high-frequency component. The signal via the low-pass filter is transmitted to the recording unit 102 and the analyzing unit 103 to be described later. Note that, in a case where the biological information measurement apparatus 10 according to the present technology includes a plurality of light receiving units 12, the low-pass filter may be provided for each light receiving unit 12.

The recording unit 102 records the information of the plurality of measurement sites extracted by the extracting unit 101. The recording unit 102 is achieved by, for example, a central processing unit (CPU), a read only memory (ROM), a random access memory (RAM), a memory such as a flash memory, a hard disk, or the like. The recording unit 102 is connected to an output end of the low-pass filter, and holds or updates the signal via the low-pass filter.

The analyzing unit 103 analyzes at least the information of the plurality of measurement sites recorded in the recording unit 102, and analyzes the biological information (for example, a state of the autonomic nerves) on the basis of a result of the analysis. The analyzing unit 103 is achieved by, for example, a central processing unit (CPU), a field programmable gate array (FPGA), or the like together with the controlling unit 104.

Specifically, the analyzing unit 103 acquires a difference between information of each measurement site (for example, each blood vessel) recorded in the recording unit 102 and information of the measurement site (for example, the blood vessel) extracted by the extracting unit 101 after the information of the measurement site is recorded in the recording unit 102. Then, the analyzing unit 103 analyzes the biological information on the basis of a result of the acquisition.

More specifically, the analyzing unit 103 acquires, for example, a correlation of a difference in information for each measurement site of the living body and discriminates a state of the living body on the basis of a result of the acquisition. Examples of the "correlation" as used herein include feature amounts such as a variance, standard deviation, difference, skewness, kurtosis, correlation, p-quantile, average value, and median value.

The analyzing unit 103 includes, for example, a subtracting unit, a correlation acquiring unit, and a state discriminating unit.

The subtracting unit is connected to the output terminal of the low-pass filter and the recording unit 102. The subtracting unit subtracts a signal (old signal) recorded (held or updated) by the recording unit 102 from a signal (new signal) transmitted via the low-pass filter after a predetermined time from the time of the recording, and outputs a result of the subtraction (difference). Note that, in a case where the biological information measurement apparatus 10 according to the present technology includes a plurality of light receiving units 12, the subtracting unit may be provided for each low-pass filter provided in each light receiving unit 12.

The correlation acquiring unit is connected to an output end of the subtracting unit. The correlation acquiring unit acquires a correlation of the result of the subtraction (difference) in the subtracting unit (calculates the feature amount described above), and outputs a result of the acquisition (result of the calculation).

The state discriminating unit is connected to an output end of the correlation acquiring unit. The state discriminating unit discriminates the state of the living body on the basis of the correlation acquired by the correlation acquiring unit.

Here, for example, a signal of each channel has a waveform corresponding to the behavior of a corresponding blood vessel. Since the behavior of a blood vessel is different for each blood vessel, a waveform of a signal acquired for each blood vessel is also different. However, a waveform of a result of the subtraction between the new and old signals of each channel described above (waveform of the difference) is information reflecting the presence or absence of a change in the state of the autonomic nerves regardless of a waveform of a signal for each blood vessel. While the autonomic nerves continuously remain in the same state, it is considered that the waveforms of the difference between the signals of each channel are approximate to each other. Hence, if the waveforms of the difference between the signals of each channel are approximate to each other, it can be estimated that the autonomic nerves continuously remain in the same state.

Therefore, in a case where the result of the acquisition (correlation) in the correlation acquiring unit, that is, the feature amount described above is, for example, less than a threshold, the state discriminating unit determines that the autonomic nerves continuously remain in the same state and outputs a result of discrimination about the state of the autonomic nerves corresponding to a result of the determination (for example, discrimination as to whether the state of the autonomic nerves is a resting state or a tension state).

Meanwhile, when the state of the autonomic nerves changes, it is considered that the waveforms of the difference between the new and old signals of each channel are not approximate to each other. Hence, if the waveforms of the difference between the new and old signals of each channel are not approximate to each other, it can be estimated that the state of the autonomic nerves has changed.

Therefore, in a case where the result of the acquisition (correlation) in the correlation acquiring unit, that is, the feature amount described above is, for example, the threshold or more, the state discriminating unit determines that the state of the autonomic nerves has changed and outputs a result of discrimination about the state of the autonomic nerves corresponding to a result of the determination (for example, discrimination as to whether the state of the autonomic nerves is a resting state or a tension state).

Note that, although a method of acquiring the difference by the subtracting unit has been described so far, a dividing unit may be included instead of the subtracting unit and a method of acquiring a result of division may be used.

Furthermore, the correlation may be acquired (the feature amount described above may be calculated) from the signal recorded in the recording unit without using subtraction or division, and a result of the acquisition (result of the calculation) may be output. In this case, the correlation includes a correlation coefficient and the like.

### 2. Biological Information Measurement Apparatus 10 According to Second Embodiment of Present Technology

Hereinafter, configurations of a biological information measurement apparatus 10 according to the present embodiment will be described. Note that the embodiment illustrates a preferred example of the present technology, and the biological information measurement apparatus 10 according to the present technology is not limited to the configurations. Furthermore, in embodiments to be illustrated below, a measurement site is not included in these embodiments.

### 2-1. Configurations of Biological Information Measurement Apparatus 10 According to Second Embodiment

The biological information measurement apparatus 10 according to the present embodiment includes a plurality of light emitting units 11 and at least one or more light receiving units 12. Hereinafter, specific configurations of the biological information measurement apparatus 10 according to the present embodiment will be described in detail. Note that the present embodiment is similar to the biological information measurement apparatus 10 according to the first embodiment described above except that the present embodiment includes the plurality of light emitting units 11 and the at least one or more light receiving units 12.

### 2-2. Configuration Example 13

A of Fig. 10 is a schematic diagram illustrating configuration example 13 of the mechanism S, and B of Fig. 10 is a schematic cross-sectional diagram taken along line Q-Q of A of Fig. 10. Configuration example 13 includes two light emitting unit 11 that irradiate the inside of a living body with light, one light receiving unit 12 that receives light scattered in the living body and acquire a biological signal, and a housing 13 including the light emitting units 11 and the light receiving unit 12, and a plurality of the light emitting unit 11 and the one light receiving unit 12 are disposed in the same straight line. Furthermore, a light shielding unit 152 that shields a part of light emitted from the plurality of the light emitting units and scattered at a plurality of measurement sites in the living body is included.

The light shielding unit 152 is disposed between the light receiving unit 12 and the measurement site and is preferably disposed in a straight line connecting the same measurement site (for example, blood vessel P2) propagated in the living body and the light receiving unit 12. With this configuration, light passing through the same measurement site (for example, blood vessel P2) is blocked by the surface of the living body, and information of different measurement sites (for example, blood vessels P1 and P3) can be acquired by the light receiving unit 12. Note that a material constituting the light shielding unit 152 is similar to the material constituting the light shielding unit 151 described above.

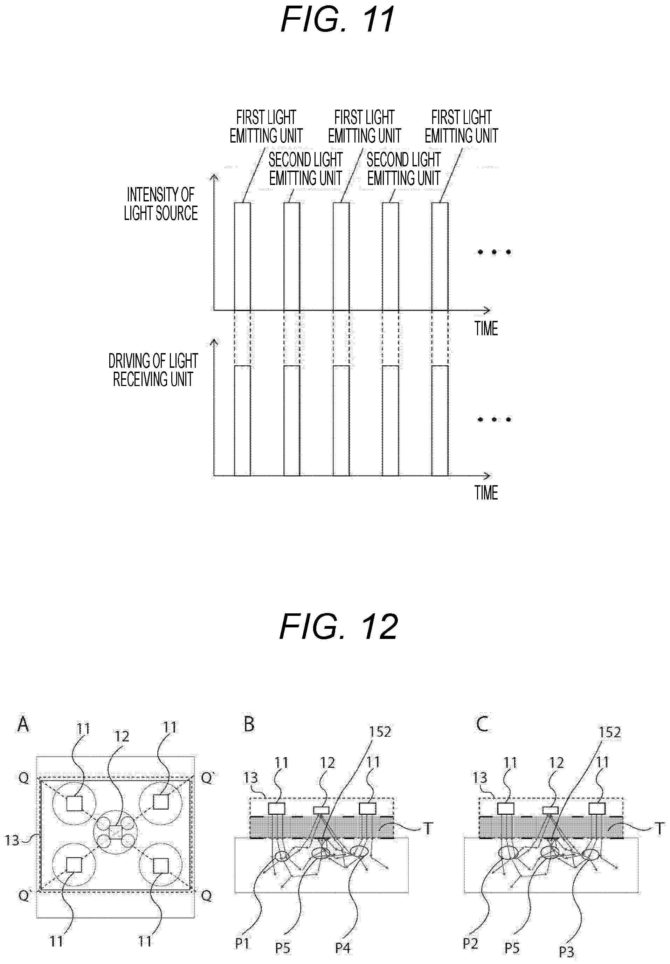

In configuration example 13, since there is one light receiving unit 12, information of different measurement sites (for example, blood vessels P1 and P3) can be acquired by time-divisionally driving the light receiving unit 12 and the two light emitting units 11 (for example, first light emitting unit and second light emitting unit) as illustrated in Fig. 11.

In the configuration example 13, a member T having a transmissive surface through which the light emitted from the light emitting unit 11 is transmitted may be further included, and the member T may be disposed, for example, between the light emitting unit 11 and the measurement sites and between the light receiving unit 12 and the measurement sites, as illustrated in B of Fig. 10. With this configuration, the light emitting unit 11 and the light receiving unit 12 can be stably disposed, and furthermore, when a blood flow rate is measured using a laser in the light emitting unit 11, a signal intensity is increased by providing a distance between the surface of the living body or the like and the light receiving unit 12. Thus, the signal intensity can be increased by increasing the distance between the surface of the living body or the like and the light receiving unit 12 with the member T interposed therebetween. Note that a material forming the member T is similar to the material of the member 14 described above.

### 2-3. Configuration Example 14

A of Fig. 12 is a schematic diagram illustrating configuration example 14 of the mechanism S, B of Fig. 12 is a schematic cross-sectional diagram taken along line Q-Q of A of Fig. 12, and C of Fig. 12 is a schematic cross-sectional diagram taken along line Q'-Q' of A of Fig. 12. Configuration example 14 includes four light emitting unit 11 that irradiate the inside of a living body with light, one light receiving unit 12 that receives light scattered in the living body and acquire a biological signal, and a housing 13 including the light emitting units 11 and the light receiving units 12, and the four light emitting units 11 are disposed in the diagonal lines of the light receiving units 12 with the one light receiving units 12 interposed between the light emitting units 11. Furthermore, a light shielding unit 152 that shields a part of light emitted from a plurality of the light emitting units and scattered at a plurality of measurement sites in the living body is included.

The light shielding unit 152 is disposed in a straight line connecting the same measurement site (for example, blood vessel P2) propagated in the living body and the light receiving unit 12. With this configuration, even in a case where the number of the light emitting units 11, light having passed through the same measurement site (for example, blood vessel P5) is blocked by the surface of the living body, and pieces of information of different measurement sites (for example, blood vessels P1, P2, P3, and P4) can be individually acquired by the light receiving unit 12.

Note that in configuration example 13 and configuration example 14, the shape of a portion where the light shielding unit 152 does not exist is substantially circular, but the present technology is not limited to this shape as long as the light having passed through the same measurement site (for example, blood vessel P2) can be blocked by the surface of the living body. Furthermore, the disposition of the light shielding unit 152 can be also freely designed.

### 3. Biological Information Measurement System According to Present Technology

Furthermore, a biological information measurement system according to the present technology includes: a light emitting device that irradiates a living body with light; and a light receiving device that receives light scattered at a plurality of measurement sites in the living body, in which the biological information measurement system includes a mechanism in which the light scattered at the plurality of measurement sites is individually detected by a plurality of light receiving units or the same light receiving unit.

The light emitting device may include, for example, a light emitting unit 11 described above. Since the light emitting unit 11 is similar to that described above, the description thereof is omitted here.

Furthermore, the light receiving device may include, for example, a light receiving unit 12 described above. Since the light receiving unit 12 is similar to that described above, the description thereof is omitted here.

Moreover, since the mechanism is similar to the mechanism S described above, the description thereof will be omitted here.

### 4. Biological Information Measurement Method According to Present Technology

The biological information measurement apparatus 10 according to the present technology can be suitably used in a biological information measurement method for measuring biological information. In the biological information measurement method according to the present technology, measurable biological information is not particularly limited, but is preferably blood vessel information of a living body. The acquisition of the blood vessel information of the living body enables, for example, identification of a state of autonomic nerves.

### REFERENCE SIGNS LIST

- 10: Biological information measurement apparatus
- 11: Light emitting unit
- 12: Light receiving unit
- 13: Housing
- 14: Member
- 151, 152: Light shielding unit
- 16: Lens
- 161: Lens light shielding unit
- 17: Diffraction grating unit
- 18: Light dividing unit
- 19: Member
- 100: Processing unit
- 101: Extracting unit
- 102: Recording unit
- 103: Analyzing unit
- 104: Controlling unit
- P1 to P5: Blood vessel
- X: Transmissive surface
- R: Refractive surface
- T: Member

## Claims

1. A biological information measurement apparatus (10) comprising:
a light emitting unit (11) configured to irradiate a living body with light;
a plurality of light receiving units (12) configured to receive light scattered at a plurality of measurement sites in the living body, wherein
the biological information measurement apparatus includes a mechanism in which the light scattered at the plurality of measurement sites is individually detected by a plurality of the light receiving units;
said mechanism comprising a lens (16) through which the light emitted from the light emitting unit is transmitted, wherein
the lens is disposed between the light emitting unit (11) and the measurement site , **characterised in that** the lens includes a lens light shielding unit (161) at a lens center part on a side where the light emitted from the light emitting unit is incident or emitted.

2. The biological information measurement apparatus according to claim 1, further comprising:
a member (14) having a transmissive surface through which the light emitted from the light emitting unit is transmitted, wherein
the member is disposed between the light emitting unit and the measurement site, and
the transmissive surface is disposed to be inclined with respect to an irradiation optical path of the light emitting unit.

3. The biological information measurement apparatus according to claim 2, wherein the plurality of light receiving units are disposed on substantially the same plane.

4. The biological information measurement apparatus according to claim 2, wherein the light emitting unit and the plurality of light receiving units are disposed on substantially the same plane.

5. The biological information measurement apparatus according to claim 1, further comprising:
a diffraction grating unit that generates diffracted light on a basis of the light emitted from the light emitting unit, wherein
the diffraction grating unit is disposed between the light emitting unit and the measurement site.

6. The biological information measurement apparatus according to claim 1, further comprising:
a light dividing unit that divides the light emitted from the light emitting unit, wherein
the light dividing unit is disposed between the light emitting unit and the measurement site.

7. The biological information measurement apparatus according to claim 1, comprising a plurality of light emitting units.

8. A biological information measurement method comprising:
a light emitting step of irradiating a living body with light emitted by a light emitting unit (11); and
a light receiving step of receiving, at a plurality of light receiving units (12), light scattered at a plurality of measurement sites in the living body, wherein
the biological information measurement method includes a mechanism in which the light scattered at the plurality of measurement sites is individually detected by the plurality of light receiving units
wherein the light emitted is transmitted through a lens (16), wherein
the lens is disposed between the light emitting unit (11) and the measurement site and includes a lens light shielding unit (161) at a lens center part on a side where the light emitted from the light emitting unit is incident or emitted.

## Patentansprüche

1. Vorrichtung zur Messung biologischer Informationen (10), umfassend:
eine Lichtemissionseinheit (11), die dazu konfiguriert ist, einen lebenden Körper mit Licht zu bestrahlen;
eine Vielzahl von Lichtempfangseinheiten (12), die dazu konfiguriert sind, Licht zu empfangen, das an einer Vielzahl von Messstellen in dem lebenden Körper gestreut wird, wobei
die Vorrichtung zur Messung biologischer Informationen einen Mechanismus einschließt, bei dem das an der Vielzahl von Messstellen gestreute Licht einzeln von einer Vielzahl von Lichtempfangseinheiten erfasst wird;
wobei der Mechanismus eine Linse (16) umfasst, durch die das von der Lichtemissionseinheit emittierte Licht übertragen wird, wobei
die Linse zwischen der Lichtemissionseinheit (11) und der Messstelle angeordnet ist, **dadurch gekennzeichnet, dass** die Linse eine Linsenlichtabschirmeinheit (161) in einem Linsenmittelteil auf einer Seite einschließt, auf die das von der Lichtemissionseinheit emittierte Licht auftrifft oder emittiert wird.

2. Vorrichtung zur Messung biologischer Informationen nach Anspruch 1, ferner umfassend:
ein Element (14), das eine durchlässige Oberfläche aufweist, durch die das von der Lichtemissionseinheit emittierte Licht übertragen wird, wobei
das Element zwischen der Lichtemissionseinheit und der Messstelle angeordnet ist, und
die durchlässige Oberfläche so angeordnet ist, dass sie in Bezug auf einen optischen Bestrahlungspfad der Lichtemissionseinheit geneigt ist.

3. Vorrichtung zur Messung biologischer Informationen nach Anspruch 2, wobei die Vielzahl von Lichtempfangseinheiten im Wesentlichen auf derselben Ebene angeordnet sind.

4. Vorrichtung zur Messung biologischer Informationen nach Anspruch 2, wobei die Lichtemissionseinheit und die Vielzahl von Lichtempfangseinheiten im Wesentlichen auf derselben Ebene angeordnet sind.

5. Vorrichtung zur Messung biologischer Informationen nach Anspruch 1, ferner umfassend:
eine Beugungsgittereinheit, die auf der Grundlage des von der Lichtemissionseinheit emittierten Lichts gebeugtes Licht erzeugt, wobei
die Beugungsgittereinheit zwischen der Lichtemissionseinheit und der Messstelle angeordnet ist.

6. Vorrichtung zur Messung biologischer Informationen nach Anspruch 1, ferner umfassend:
eine Lichtteilungseinheit, die das von der Lichtemissionseinheit emittierte Licht teilt, wobei
die Lichtteilungseinheit zwischen der Lichtemissionseinheit und der Messstelle angeordnet ist.

7. Vorrichtung zur Messung biologischer Informationen nach Anspruch 1, umfassend eine Vielzahl von Lichtemissionseinheiten.

8. Verfahren zur Messung biologischer Informationen, umfassend:
einen Lichtemissionsschritt zum Bestrahlen eines lebenden Körpers mit Licht, das von einer Lichtemissionseinheit (11) emittiert wird; und
einen Lichtempfangsschritt zum Empfangen, an einer Vielzahl von Lichtempfangseinheiten (12), von Licht, das an einer Vielzahl von Messstellen in dem lebenden Körper gestreut wird, wobei
das Verfahren zur Messung biologischer Informationen einen Mechanismus einschließt, bei dem das an der Vielzahl von Messstellen gestreute Licht einzeln von der Vielzahl von Lichtempfangseinheiten erfasst wird,
wobei das emittierte Licht durch eine Linse (16) übertragen wird, wobei
die Linse zwischen der Lichtemissionseinheit (11) und der Messstelle angeordnet ist und eine Linsenlichtabschirmeinheit (161)
an einem Linsenmittelteil auf einer Seite einschließt, auf die das von der Lichtemissionseinheit emittierte Licht auftrifft oder emittiert wird.

## Revendications

1. Appareil de mesure d'informations biologiques (10) comprenant :
une unité d'émission de lumière (11) configurée pour irradier un corps vivant avec de la lumière ;
une pluralité d'unités de réception de la lumière (12) configurées pour recevoir la lumière diffusée sur une pluralité de sites de mesure dans le corps vivant, dans lequel
l'appareil de mesure des informations biologiques comporte un mécanisme dans lequel la lumière diffusée sur la pluralité de sites de mesure est détectée individuellement par une pluralité d'unités de réception de la lumière ;
ledit mécanisme comprend une lentille (16) à travers laquelle la lumière émise par l'unité émettrice de lumière est transmise, dans lequel
la lentille est disposée entre l'unité émettrice de lumière (11) et le site de mesure, **caractérisé en ce que** la lentille comporte une unité de protection contre la lumière (161) au niveau d'une partie centrale de la lentille sur un côté où la lumière émise par l'unité émettrice de lumière est incidente ou émise.

2. Appareil de mesure d'informations biologiques selon la revendication 1, comprenant en outre :
un élément (14) ayant une surface transmissive à travers laquelle la lumière émise par l'unité émettrice de lumière est transmise, dans lequel
l'élément est disposé entre l'unité d'émission de lumière et le site de mesure, et
la surface transmissive est inclinée par rapport à un trajet optique d'irradiation de l'unité émettrice de lumière.

3. Appareil de mesure d'informations biologiques selon la revendication 2, dans lequel la pluralité d'unités de réception de la lumière sont disposées sensiblement sur le même plan.

4. Appareil de mesure d'informations biologiques selon la revendication 2, dans lequel l'unité émettrice de lumière et la pluralité d'unités réceptrices de lumière sont disposées sensiblement sur le même plan.

5. Appareil de mesure d'informations biologiques selon la revendication 1, comprenant en outre :
un réseau de diffraction qui génère une lumière diffractée sur la base de la lumière émise par l'unité d'émission de lumière, dans lequel
le réseau de diffraction est placé entre l'unité d'émission de lumière et le site de mesure.

6. Appareil de mesure d'informations biologiques selon la revendication 1, comprenant en outre :
une unité de division de la lumière qui divise la lumière émise par l'unité d'émission de lumière, dans lequel
l'unité de division de la lumière est disposée entre l'unité d'émission de lumière et le site de mesure.

7. Appareil de mesure d'informations biologiques selon la revendication 1, comprenant une pluralité d'unités émettrices de lumière.

8. Procédé de mesure d'informations biologiques, comprenant :
une étape d'émission de lumière consistant à irradier un corps vivant avec de la lumière émise par une unité d'émission de lumière (11) ; et
une étape de réception de lumière consistant à recevoir, au niveau d'une pluralité d'unités de réception de la lumière (12), la lumière diffusée au niveau d'une pluralité de sites de mesure dans le corps vivant, dans lequel
le procédé de mesure d'informations biologiques comporte un mécanisme dans lequel la lumière diffusée sur la pluralité de sites de mesure est détectée individuellement par la pluralité d'unités de réception de lumière
dans lequel la lumière émise est transmise à travers une lentille (16), dans lequel
la lentille est disposée entre l'unité d'émission de lumière (11) et le site de mesure et comporte une unité de protection contre la lumière de la lentille (161)
au centre de la lentille, du côté où la lumière émise par l'unité d'émission de lumière est incidente ou émise.
